# EUROPEAN PATENT APPLICATION

(11) **EP 4 548 762 A2**
(43) Date of publication of application: **07.05.2025**
(21) Application number: 25159442.0
(22) Date of filing: 01.03.2023
(51) Int. Cl.: A01N 1/00, A61D 19/02

(54) **METHOD OF CRYOPRESERVING BIOLOGICAL MATERIAL AND RELATED APPARATUS**

(30) Priority: 04.03.2022 NO 20220277
(62) Divisional of application: 23708769.7
(71) Applicant: Cryogenetics AS, 2317 Hamar (NO)
(72) Inventor: WOLLA, Steffen, 2407 Elverum (NO); GREVLE, Inger Synnøve, 2316 Hamar (NO)
(74) Representative: Onsagers AS

(57) **Abstract**

The disclosure relates to a method of cryopreserving biological material in a plurality of dimensionally stable containers (100), each having two parallel walls connected to each other along a substantially part of their periphery and defining between them a storage space having a uniform thickness. The method comprises the steps of, in a housing or cabinet of a cryopreservation apparatus: at least partially filling the storage space with the biological material via an opening in the containers; closing the opening enclosing the biological material in the containers; racking the containers in substantially parallel planes; and freezing the biological material enclosed in the containers to a cryogenic temperature. According to the method, the step of freezing the biological material includes bringing the racked containers and the biological material enclosed therein to a first reservoir (356) holding a first freezing agent at a first temperature and fully immersing the containers in the first freezing agent and circulating the first freezing agent between the racked containers. The disclosure also relates to a related apparatus.

## Description

The present disclosure relates to a method and an apparatus for cryopreserving biological material, in particular fish milt.

In particular, the present disclosure relates to a method and an apparatus for cryopreserving biological material in containers comprising two substantially parallel walls connected to each other along a substantially part of their periphery and defining between them a storage space having a substantially uniform thickness. An example of such a container is disclosed in WO2009151339A2.

Cryopreserving biological material in containers comprising two substantially parallel walls connected to each other along a substantially part of their periphery and defining between them a storage space having a substantially uniform thickness, allows the freezing curve or freezing profile of the biological material to be controlled in a precise manner. This, in turn, provides for a large survival rate of the cryopreserved biological material.

The present invention relates to improvements in cryopreserving biological material in such containers.

An object of the present disclosure is to provide an improved method of cryopreserving biological material.

Another object is to provide an improved apparatus for cryopreserving biological material.

A further object is to provide a method and an apparatus allowing controlled freezing of biological material.

A further object is to provide a method and an apparatus allowing controlled batch-wise freezing of biological material.

Yet a further object is to provide a method and an apparatus allowing a large through-put of cryopreserved containers.

The claimed invention is defined in the appended claims.

According to one aspect, the present disclosure provides a method of cryopreserving biological material in a plurality of dimensionally stable containers, each container comprising two substantially parallel walls connected to each other along a substantially part of their periphery and defining between them a storage space having a substantially uniform thickness, wherein the method comprises the steps of:
- at least partially filling the storage space with the biological material via an opening in the container;
- closing the opening enclosing the biological material in the containers; and
- freezing the biological material enclosed in the containers to a cryogenic temperature.

The method comprises the step of racking the containers in substantially parallel planes and, during said step of freezing the biological material, bringing the racked containers and the biological material enclosed therein to a first reservoir holding a first freezing agent or fluid at a first temperature, fully immersing the racked containers in the first freezing agent and circulating the first freezing agent between the racked containers.

The step of racking the containers may comprise racking the containers in substantially vertical planes, and the containers may be racked with a mutual distance of at least 5 mm or, more preferably with a mutual distance of at least 10 mm. This allows the first freezing agent access to the parallel walls of each container, which access ensures controlled freezing of the biological material. However, it has been found that racking the containers with a mutual distance which is beyond approximately 15 mm does not further increase controllability of the freezing process to any large extent. Therefore, the containers may advantageously be racked with a mutual distance within the range of 0.5 to 15 mm or, more preferably, within the range of 10 to 15 mm.

The method may further comprise the steps of:
- keeping the racked containers submerged in the first freezing agent for a first period of time;
- disinvolving the racked containers from the first freezing agent, i.e. raising the rack holding the containers from the first freezing agent such that the racked containers are relieved from involvement with the first freezing agent or, in other words, are no longer immersed in the first freezing agent;
- bringing the racked containers from the first reservoir to a second reservoir holding a second freezing agent or fluid at a second temperature being lower than the first temperature and immersing the racked containers in the second freezing agent; and
- keeping the racked containers submerged in the second freezing agent for a second period of time.

By using this two-step freezing process, even more controlled freezing of the biological material enclosed in the storage space having the substantially uniform thickness can be achieved.

One of said first and second temperatures may be non-cryogenic, i.e. having a temperature higher than -180°C. For example, it may be preferable that the first temperature is a non-cryogenic temperature, i.e. a temperature higher than -180°C, and the second temperature a cryogenic temperature, i.e. a temperature lower than -180°C.

The first and second temperatures may be arranged to vary during said first and second period of time, thus exposing the container to decreasing and/or increasing temperatures when immersed in the first and second freezing agent. However, it may be preferable that the first and second temperatures are kept substantially constant during the first and second period of time, respectively.

The first period of time may be within the range of 60 s to 300 s and the first temperature may be kept constant within the range of -20°C to -90°C during the first period of time.

Alternatively, the first period of time may be within the range of 60 s to 3600 s and the first temperature may be arranged to continuously decrease from e.g. +25°C to e.g. -90°C during the first period of time.

The second period of time may be any one of: longer than the first period of time; shorter than the first period of time; and equal to the first period of time. Preferably, however, the second period of time may be any one of: within the range of 10 s to 240 s, at least 10 s, at least 30 s, at least 60 s, and at least 120 s.

The first freezing agent or fluid may be a freezing liquid or a freezing vapour. Likewise, the second freezing agent may be a freezing liquid or a freezing vapour

The first freezing agent or fluid may be liquid oil or liquid alcohol, e.g. a low molecular weight alcohol, e.g. a linear and branched monohydric aliphatic C1 to C6 alcohol, including methanol, ethanol, and 1-propanol. According to one embodiment, the first freezing agent or fluid may be isopropanol, e.g. an isopropyl alcohol (IUPAC name propan-2-ol and also called 2-propanol; chemical formula CH3CHOHCH3).

The second freezing agent may be liquid nitrogen and/or nitrogen vapour.

After said step of freezing the biological material, the method may comprise the step of packaging the containers in boxes or packages for subsequent cryogenic transport and/or storage.

According to one aspect, the present disclosure provides a cryopreservation apparatus for batch-wise cryopreservation of biological material in dimensionally stable containers, each container comprising two substantially parallel walls connected to each other along a substantially part of their periphery and defining between them a storage space having a substantially uniform thickness, the apparatus comprising:
- a filling station configured for filling the containers with the biological material;
- a sealing station configured for sealing the containers enclosing the biological material in the containers; and
- a freezing station configured for freezing the biological material enclosed in the containers to a cryogenic temperature.

The cryopreservation apparatus comprises a racking station arranged downstream of the sealing station and upstream of the freezing station for racking a plurality of said containers in a rack in substantially parallel planes. The freezing station comprises a first reservoir holding a first freezing agent or fluid at a first temperature, the first reservoir being configured for receiving the racked containers fully immerged in the first freezing agent. The freezing station also comprises a pump configured for circulating the first freezing agent between the immersed, racked containers.

The racking station may be configured to rack the containers in substantially vertical planes. For example, as previously discussed, the racking station may be configured to rack the containers in substantially vertical planes with a mutual distance of any one of: at least 5 mm; at least 10 mm; within a range of 5 to 15 mm and within a range of 10 to 15 mm.

The first reservoir may comprise a distributor configured for evenly distributing the circulated first freezing agent between the racked containers.

The cryopreservation apparatus may comprise a second reservoir holding a second freezing agent or fluid at a second temperature being lower than the first temperature, wherein the second reservoir is configured for receiving the container fully immerged in the second freezing agent after the container first having been fully submerged in the first freezing agent.

As previously discussed, the first temperature may be a non-cryogenic temperature and the second temperature a cryogenic temperature. Also as previously discussed, the first freezing agent may be liquid alcohol and the second freezing agent liquid nitrogen and/or nitrogen vapour.

The cryopreservation apparatus may comprise a packaging station arranged downstream of the freezing station, which packaging station may be configured for packaging the cryogenically frozen containers in boxes or packages for subsequent cryogenic transport and/or storage.

The cryopreservation apparatus may comprise a housing or cabinet in which the filling station, the sealing station, the racking station, the freezing station and, optionally, the packaging station, is housed. The cabinet may be configured to hold a temperature within the range of ±0°C to +10°C, e.g. within the range of +3°C to +5°C. To this end, the apparatus may comprise a cooling system, e.g. a vapour-compression refrigeration system and/or an air circulation system, e.g. comprising a fan, configured to circulate cooled air in the housing or cabinet.

The biologic material suitable to be cryopreserved according to the present disclosure is for example semen, embryos, eggs, gametes etc. The method and apparatus according to the present disclosure is particularly suitable for cryopreservation of spermatozoa collected from aquatic species. In this context, aquatic species are any organism living in water and wherein reproduction is performed by the joining of spermatozoa and egg from a male and female animal, respectively, herein organisms employing oviparous, ovoviviparous or viviparous reproduction. The method and apparatus according to the present disclosure allows packaging of male gametes (sperm) collected from marine, anadromous and freshwater fish.

Non-limiting examples of oviparous fish species are, inter alia, Salmonidae, such as Atlantic salmon (*Salmo salar* L.) and rainbow trout (*Onchorhyncus mykiss*), Gadidae, such as Atlantic cod (*Gadus morhud*) and haddock (*Melanogrammus aeglefinus*) and Cichlidae, such as Nile tilapia (*Oreochromis niloticus niloticus*). Non-limiting examples of ovoviviparous fish include inter alia Poecilidae, such as guppy (*Poecilia reticulata*) and Squatinidae, such as angel sharks (*Squatina* spp.). Non-limiting examples of viviparous fishes includes coelacanths (*Latimeria chalumnae*), Goodeidae (splitfins), Embiotocidae (surf-perches) and Carcharhinidae (requiem sharks).

However, the method and apparatus according to the present disclosure is not limited for cryopreserving biological material from fish species, but is also suitable for cryopreservation of biological material from other water-dwelling organisms such as Crustacea, inter alia crabs, lobsters, crayfish, shrimps, shellfish and barnacles, and Mollusca, inter alia gastropods such as abalone, cephalopods such as octopus, and bivalves such as scallops, clams and oysters.

The method and apparatus according to the present disclosure is also suitable for cryopreservation of biological material from algae, including but not limited to the class Phaeophyceae (brown algae), e.g. from the order Fucales or Laminariales (kelp). In particular, the method and apparatus according to the present disclosure is suitable for cryopreserving zoospores, gametophytes, gametes, eggs, zygotes or sporophytes of such organisms.

Said parallel arrangement of the walls of the containers provides an evenly distribution of the biological material to be frozen. The distance separating the walls decides the thickness of the layer of biologic material in said storage space: Thus, a small distance provides a thin layer of biological material and allows a uniform freezing of the content. The walls may have any shape and may be straight or not, i.e. wavy, as long as they are substantially parallel and provide an evenly distribution of a thin layer of the biological material.

The storage space may typically have a substantially uniform thickness within the range of 0.5 to 3.0 mm. However, to allow even more controlled freezing of the biological material, in particular when the material is spermatozoa collected from aquatic species, it may be preferable that the substantially uniform thickness of the storage space of the container is within the range of 0.8 mm to 2.2 mm.

The containers may for example be made from polyethylene terephthalate glycol (PETG), polyethylene (PE), thermoplastic polyester (PET), polyvinyl chloride (PVC) or high securing ionomeric resin (IR), polyethylene glycol (PEG), PE, or any combinations thereof. The walls may have a thickness within the range of 0.2 mm to 0.4 mm.

Above-discussed preferred and/or optional features of each aspect of the invention may be used, alone or in appropriate combination, in the other aspects of the invention.

Following drawings are appended to facilitate the understanding of the invention:
Fig. 1 is a perspective view of a container for cryopreserving biological material;
Fig. 2 is a rear view of the container shown in Fig. 1;
Fig. 3 is a front view of the container shown in Fig. 1;
Fig. 4 is a first side view the container shown in Fig. 1, the second side view being identical thereto;
Fig. 5 is a bottom view of the container shown in Fig. 1;
Fig. 6 is a top view of the container shown in Fig. 1;
Fig. 7 is a sectional view of the container shown in Fig. 1 as viewed between lines XII and XII' in Fig. 2;
Fig. 8 is a schematic depiction of a cryopreservation apparatus according to one embodiment of the invention;
Fig. 9 is a schematic illustration of a rotatable conveyor of the cryopreservation apparatus according to Fig. 8
Fig. 10 shows a cryopreservation apparatus according to one embodiment of the invention;
Figs. 11-13 are detailed views of the cryopreservation apparatus according to Fig. 10;
Fig. 14 is a detailed view of a rack of the cryopreservation apparatus;
Fig. 15 is a detailed view of a rack carrying racked containers; and
Figs 16 to 18 illustrate a distributor arranged for evenly distributing a freezing agent between racked containers.

In the drawings, like reference numerals have been used to indicate common parts, elements or features unless otherwise explicitly stated or implicitly understood by the context.

In the following, one or more specific embodiments of the invention will be described in more detail with reference to the drawings. However, it is specifically intended that the invention is not limited to the embodiments and illustrations contained herein but includes modified forms of the embodiments including portions of the embodiments and combinations of elements of different embodiments as come within the scope of the following claims. It should be appreciated that in the development of any such actual implementation, as in any engineering or design project, numerous implementation- specific decisions must be made to achieve the developer's specific goals, such as compliance with system and/or business-related constraints, which may vary from one implementation of the invention to another. Moreover, it should be appreciated that such a development effort might be complex and time consuming, but would nevertheless be a routine undertaking of design, fabrication and manufacture for the skilled person having the benefit of this disclosure.

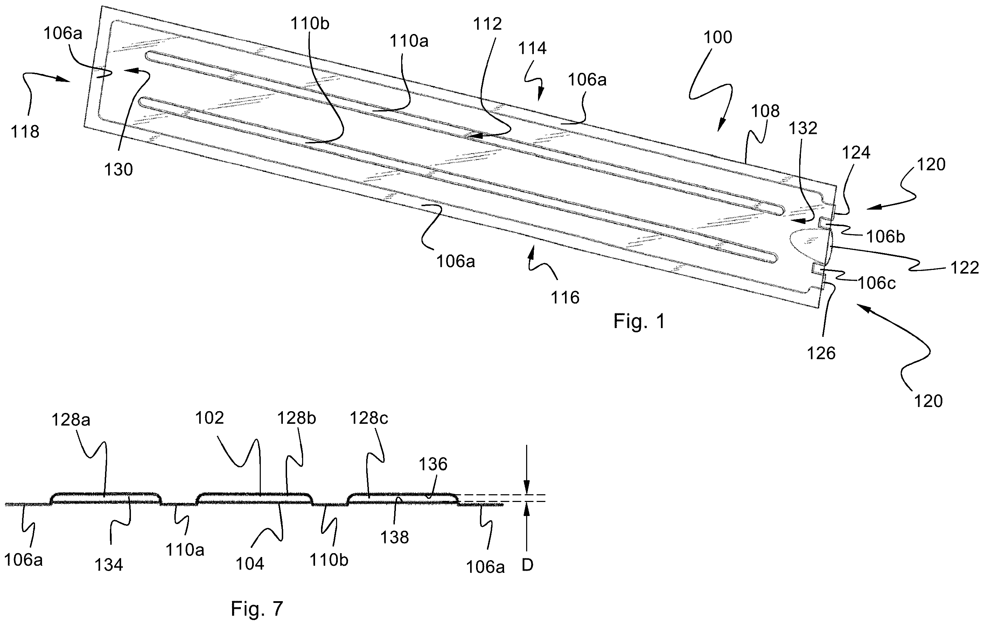

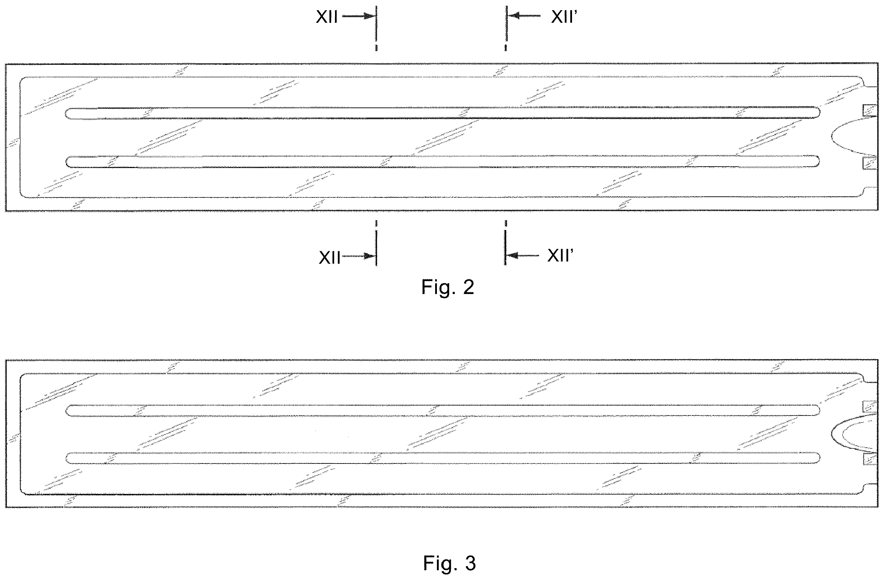

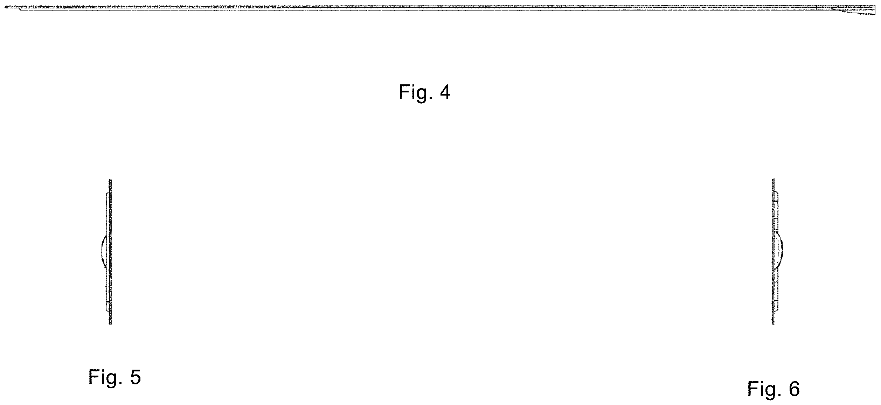

Figs. 1-7 show an embodiment of a dimensionally stable container 100 for cryopreserving biological material.

The container 100 comprises a first, front wall 102 and a second, back wall 104 made from two substantially parallel sheets of material. The walls 102, 104 are connected to each other along a substantial part of their periphery 108 and also in a central area 112 of the container 100.

In this particular embodiment, the walls 102, 104 are quadrilateral and elongated, each displaying first and second longitudinal sides 114, 116, a bottom side 118 and a top side 120. The longitudinal sides 114, 116 are parallel or substantially parallel, as are the bottom and top sides 118, 120. Consequently, the container 100 has a generally rectangular shape.

The walls 102, 104 are uninterruptedly connected to each other in a substantially U-shaped connection zone 106a extending along the longitudinal sides 114, 116 and also along the bottom side 118. Along the top side 120, the walls are intermittently connected to each other in connection zones 106b and 106c, thereby forming an opening 122 for filling the container and two air-evacuation openings 124, 126 arranged to allow air to escape the container during filling. In this embodiment the opening 122 is shaped as a funnel in order to facilitate filling of the container 100.

In the central area 112, the walls 102, 104 are connected to each other in two central connection zones 110a, 110b, each extending rectilinearly and uninterruptedly in the longitudinal direction of the container 100 parallel to the longitudinal sides 114, 116, thereby defining three parallel channel part-volumes 128a, 128b, 128c in the container 100. The connection zones 110a and 110b reach over a substantial length along the longitudinal central axis of the container. However, they terminate prior to reaching the bottom and top sides 118, 120, thereby allowing the part-volumes 128a, 128b, 128c to communicate with each other via a bottom part-volume 130 and a top part-volume 132, each extending transversely over the container 100 between the longitudinal sides 114 and 116.

The connection zones 106b and 106c are aligned with the connection zones 110a and 110b, thus positioning the opening 122 in line with part-volume 128b, i.e. the central part-volume of said three parallel channel part-volumes 128a, 128b, 128c.

The top part-volume 132 communicates with the openings 122, 124, 126, thereby allowing a storage space 134 of the container 100, formed by the part-volumes 128a-c, 130 and 132, to be filled (or partly filled, if so desired) via opening 122. During filling of the container, the configuration of the container 100 allows an injection nozzle to be inserted into part-volume 128b via opening 122, thereby allowing the biological material to flow into part-volume 128b and subsequently into part-volumes 128a and 128c via the bottom part-volume 130. In this way, any air in the container will be pushed towards the openings 124 and 126, thus ensuring that little or no air is left in the container after filling.

In the connection zones 106a-106c, 110a and 110b, the front and back walls 102, 104 of the container are connected to each other, thus having no distance between them. At the part-volumes 128a-c, 130 and 132, however, the walls 102, 104 are arranged parallel to one another with a mutual distance D between inner, opposing surfaces 136, 138 of the front and back walls 102, 104 (see Fig. 7). Consequently, the distance D defines the thickness of the storage space 134.

The distance D is substantially uniform throughout the storage space 134, thus allowing biological material filled in the storage space 134 to be frozen in a controlled and uniform manner. The chosen distance D of the container 100 will depend on the desired cooling rate of the biological material during the freezing process. Generally, however, the distance D may be within the range of approximately 0.8-2.2 mm.

The material from which the walls 102, 104 of the container 100 is made may be polyethylene terephthalate glycol (PETG), polyethylene (PE), thermoplastic polyester (PET), polyvinyl chloride (PVC) or high securing ionomeric resin (IR), polyethylene glycol (PEG), PE, or any combinations thereof.

In one embodiment of the container 100, at least one of the walls 102, 104 may be made of PET and the inner surface of said walls may be coated with PE.

In still another embodiment, one of the walls 102, 104 may be made of PET and the other opposite sheet/wall may be made of PE. Using PET and PE in the production of the container allows the container to be sealed by pulse welding subsequent to filling the container with biological material.

The thickness of the sheets from which the container 100 is made may be within the range of 0.2-0.4 mm. This will allow efficient heat transfer through the walls 102, 104 during the freezing process. This will also allow the walls 102, 104 to flex somewhat during the freezing process, thus allowing some expansion of the biological material during the liquid-to-solid phase transition. However, the arrangement of the connection zones 110a and 110b will keep the dimensional integrity of the container 100 intact throughout the filling and freezing process.

In the present embodiment, the breadth of the container 100 is approximately 55 mm and the length is approximately 315 mm. The breadth of the connection zones 106a, 110a and 110b is approximately 5 mm and the breadth of each channel part-volume 128a, 128b, 128c is approximately 12 mm. Consequently, in the present embodiment the volume of the storage space 134 can generally be varied within the range of approximately 10-20 cm³, depending on the thickness of the storage space, i.e. the distance D.

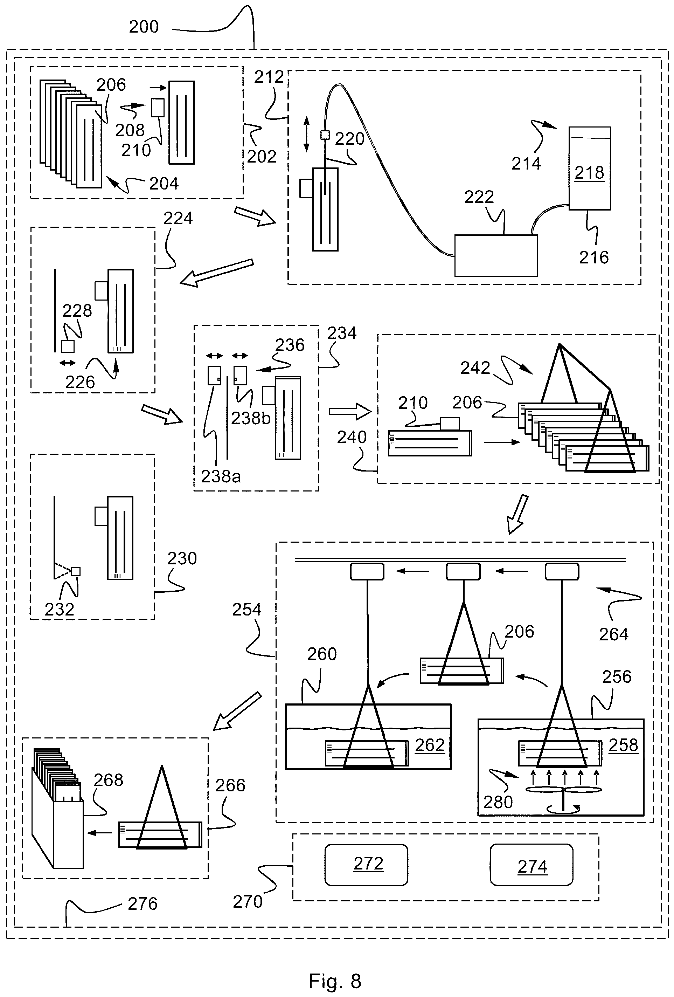

Fig. 8 is a schematic representation of a cryopreservation apparatus 200 for cryopreserving biological material in dimensionally stable, flat containers, each comprising two substantially parallel walls connected to each other along a substantial part of their periphery and defining between them a storage space having a substantially uniform thickness. The containers may advantageously be of the type discussed above with reference to Figs. 1-7.

The apparatus 200 comprises a pick-up station 202 having a storage 204 of empty containers 100 from which a conveyor comprising a gripping device 208 having a gripping member 210, e.g. a suction cup, can pick-up the containers.

The apparatus 200 also comprises a filling station 212 configured for filling the containers 100 with biological material to be cryopreserved. The filling station 212 comprises a storage 214 holding the biological material. In the present embodiment, the storage 214 comprises a tank 216 in which the biological material 218 is held. The filling station 212 also comprises a nozzle or cannula 220 which is configured for being inserted in a filling opening in a container and a pump 222 configured for supplying biological material 218 to the cannulas 220 from the storage 214.

The apparatus 200 may comprise an ID marking station 224 configured for marking the containers 100 with an ID marking or tag 226, e.g. a QR code. The ID tag may identify the individual container, its content, date and time of filling or other desirable information. In the present embodiment, the ID marking station 224 comprises a printing device 228 configured for printing the ID marking 226 on a container.

In Fig. 8 the ID marking station 224 is arranged downstream of the filling station 212. However, in other embodiments the ID marking station may be arranged at other positions of the production flow, e.g. upstream of the filling station 212 or further downstream than depicted in Fig. 8.

In still other embodiments, the ID marking station may be omitted.

The apparatus 200 may comprise an ID scanning station 230 configured for scanning the containers 100 for an ID tag, e.g. to verify the ID tag applied to the container in the ID marking station 224. In the present embodiment, the ID scanning station 230 comprises an optical scanning device 232 configured for scanning a container for an ID marking.

The apparatus 200 further comprises a sealing station 234 configured for sealing a container 100 when it has been filled with the biological material in the filling station 212. In the present embodiment, the sealing station 234 comprises a sealing device 236 having sealing members 238a, 238b which are configured to be brought into contact with the container to seal the opening or openings through which the biological material was filled. If the container 100 is of the type previously discussed with reference to Figs. 1-7, the sealing elements 238a, 238b may for example be configured to provide a weld or seam across the openings 122, 124, 126 by fusing the front 102 and back 104 walls in the area of the openings 122, 124, 126, e.g. by pulse welding.

The apparatus 200 also comprises a racking station 240 configured for racking a plurality of filled and sealed containers 100 in a rack 242.

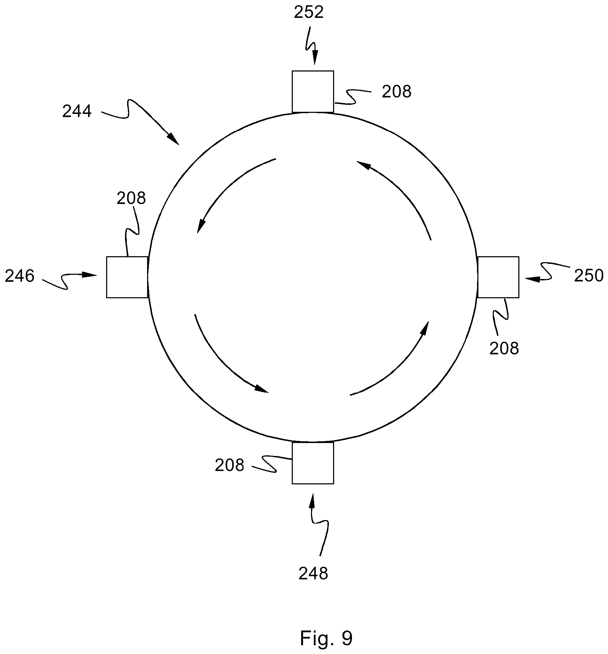

The filling, marking, sealing and racking of the containers 100 may be effectuated by a rotatable conveyor 244 comprising four gripping devices 208, each being configured for releasably gripping and holding a container, e.g. using the above-discussed gripping member 210 (see Fig. 9). The conveyor 244 is configured to rotate each gripping device 208a-208d sequentially between a first position 246, a second position 248, a third position 250 and a fourth position 250.

The pick-up station 202 is arranged at the first position 246 and at this position the gripping device 208a-208d is configured to pick up an empty container 100 from the storage 204.

After having picked up a container 100 at the first position 246, the conveyor 244 is rotated a quarter of a revolution, bring the container to the second position 248. At the same time, the next gripping device in the sequence of gripping devices is brought to the first position 246 to pick up a new container from the storage 204. The filling station 212 is arranged at the second position 248 and at this position the cannula 220 is brought into the opening of the container and the container is filled with the biological material. The ID marking station 234 may also be arranged at the second position 248 and the printing device 228 may be configured to print the ID marking 226 on the container during the filling of the container.

After the container has been filled with the biological material and, if the ID marking station 234 is implemented, the ID marking has been printed on the container, the conveyor 244 is rotated yet a quarter of a revolution, bring the filled container to the third position 250. The sealing station 234 is arranged at the third position 250 and at this position the sealing device 236 is configured to seal the container in a manner that has been previously discussed. The ID scanning station 230 may also be arranged at the third position 250 and the scanning device 232 may be configured to scan the container for ID markings, e.g. to verify the ID marking 226 printed on the container at the second position 248.

After the container has been sealed, the conveyor 244 is rotated yet a quarter of a revolution, bring the sealed container to the fourth position 252. The racking station 240 is arranged at the fourth position 252 and at this position the gripping device 208 is configured to position the sealed container in the rack 242. The conveyor 244 is then rotated yet a quarter of a revolution, bringing the now empty gripping device 208 back to the pick-up position 246 to pick up another empty container and repeat the cycle.

The apparatus 200 further comprises a freezing station 254 configured for bringing the biological material enclosed in the sealed containers to a cryogenic temperature, i.e. to a temperature below -180°C. The freezing station 254 comprises a first reservoir 256 holding a first freezing agent or fluid 258 at a first temperature T1, which may be non-cryogenic, and a second reservoir 260 holding a second freezing agent or fluid 262 at a second temperature T2, which may be cryogenic. The freezing station 254 also comprises a rack conveyor 264 configured for bringing the rack 242 and the rack-mounted containers from the racking station 240 to the first reservoir 256, immersing the rack mounted containers in the first freezing agent 258 and keeping the containers submerged therein for a first period of time t1, disinvolving the rack-mounted containers from the first freezing agent 258, bringing the rack-mounted containers to the second reservoir 260 and immersing the containers in the second freezing agent 262 and keeping the containers submerged therein for a second period of time t2.

According to one embodiment, the first freezing agent 258 is a freezing liquid kept at a constant or substantially constant temperature within the range of -20°C to -90°C, and the first period of time t1, i.e. the period of time the rack-mounted containers are kept submerged in the first freezing agent 258, is within the range of 60s to 300s.

According to an alternative embodiment, the first freezing agent 258 is a freezing liquid kept at a continuously decreasing temperature within the range of +25°C to -90°C, preferably within ±0°C to -90°C, and the first period of time t1 is within the range of 60s to 3600s.

The first freezing liquid may, according to one embodiment, be an oil. According to another embodiment, the first freezing liquid may be a linear and branched monohydric aliphatic C1 to C6 alcohol or a mixture thereof.

According to one embodiment, the second freezing agent 262 is nitrogen vapour. According to this embodiment, the second reservoir 260 contains liquid nitrogen and the rack-mounted containers are immersed into the nitrogen vapour but not into the liquid nitrogen. According to an alternative embodiment, the rack-mounted containers are immersed into the liquid nitrogen after first having been immersed in nitrogen vapour and, consequently, the second freezing agent 262 is liquid nitrogen and nitrogen vapour.

The second period of time t2, i.e. the period of time the rack-mounted containers are kept submerged in the second freezing agent 262, may be shorter, equal to or longer than the first period of time t1. However, in order to ensure that the biological material enclosed in the containers 100 are frozen to a cryogenic temperature, the second period of time t2 should preferably be at least 10 s, more preferably at least 30 s, more preferably at least 60 s, and even more preferably at least 120 s. Typically, the second period of time t2 may be within the range of 10 s to 240 s.

In the racking station, the containers 100 are advantageously stacked in the rack 242 in a side-by-side configuration in parallel and vertical stacking planes with the longitudinal sides 114, 116 of the containers 100 in a horizontal orientation. Advantageously, the distance between adjacent stacking planes is at least 5 mm, preferably at least 10 mm, thus allowing the freezing agents 258, 262 immediate access to the outside surfaces of the front and back walls 102, 104 of each container 100, thereby ensuring efficient heat transfer from each container and the biological material enclosed therein. However, it may be preferable that the mutual distance between the containers does not exceed approximately 15 mm, since this increasing the distance between the containers beyond this measure does not necessarily increase heat transfer from the containers but merely increases the volume occupied by the racked containers.

Each rack 242 can hold a plurality of containers, e.g. 10 to 20 containers, thus allowing batch-wise freezing of containers 100.

In the first reservoir 256, the first freezing agent is circulated between the racked containers 100. In Fig. 8 this is schematically illustrated by a rotor 280 forcing the first freezing agent 258 vertically upwards in between the vertically arranged containers in the rack. It is to be understood, however, that forced cooling of the racked containers may be achieved by other means, e.g. by a pump and forcing the first freezing agent to circulate between the containers.

When disinvolving the rack-mounted containers from the first freezing agent 258, the rack 242 is advantageously raised from the first reservoir at a vertical speed not exceeding 2 cm/s, and preferably not exceeding 1 cm/s. This will allow the surface tension of the first freezing agent 258 to draw and retain any freezing agent droplets formed on the surfaces of the rack 242 and the containers 100 stacked therein, thereby mitigating the problem of the first freezing agent 258 being transferred to the second reservoir 260 together with the rack 242 and the containers and contaminating the second freezing agent 262.

The apparatus 200 also comprises a packaging station 266 configured for packaging the cryogenically frozen containers in boxes or packages 268 for subsequent cryogenic transport and/or storage. This packaging operation may advantageously be carried out whilst the containers are submerged in the second freezing agent, e.g. submerged in nitrogen vapour.

The apparatus 200 further comprises a control unit 270 configured for controlling the various sub-systems of the apparatus, e.g. the above-discussed stations 202, 212, 224, 230, 240, 254 and 266, which control unit advantageously comprises a central processing unit (CPU) 272 and an input/output interface 274 allowing an operator to monitor and/or interact with the apparatus 200, e.g. to input program instructions.

The apparatus 200 may further comprise a cabinet 276 in which the sub-systems are enclosed, thus allowing at least some of the disclosed sub-systems to be kept at a controlled environment, e.g. at a controlled temperature with the range of ±0°C to +10°C, preferably within the range of +3°C to +5°C. To this end, the apparatus may comprise a cooling system, e.g. a vapour-compression refrigeration system (not disclosed).

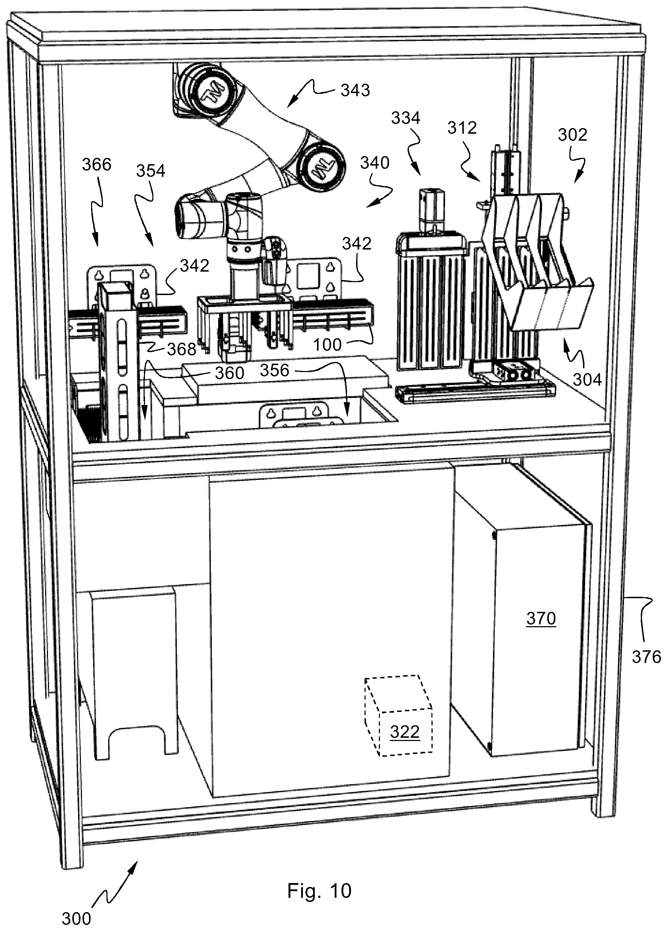

Figs. 10-13 discloses a second embodiment of a cryopreservation apparatus 300 for cryopreserving biological material, in particular spermatozoa collected from aquatic species, in dimensionally stable, flat containers, each comprising two substantially parallel walls connected to each other along a substantial part of their periphery and defining between them a storage space having a substantially uniform thickness. The containers may advantageously be of the type discussed above with reference to Figs. 1-7.

The apparatus 300 comprises a pick-up station 302 including a storage 304 for empty containers. In the present embodiment, the storage 304 comprises three cartridges which are disclosed without containers in Fig. 10.

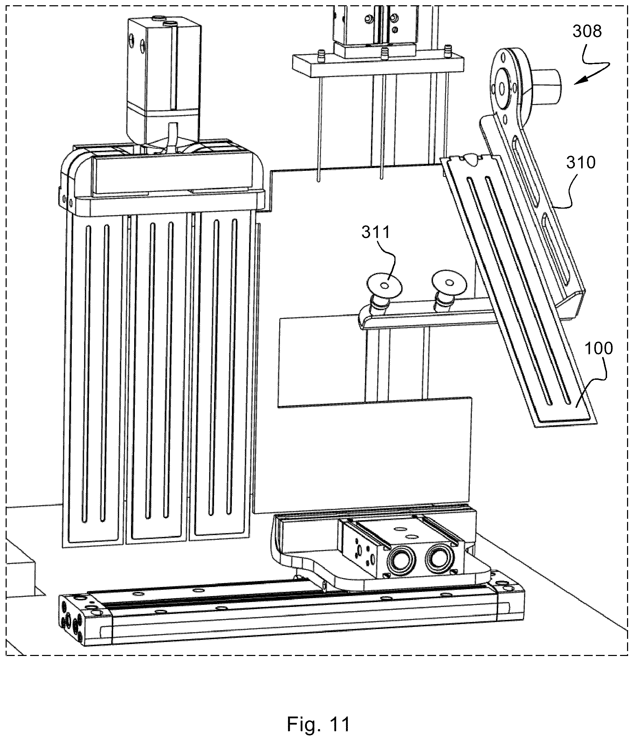

The pick-up station 302 comprises a gripping device 308 (see Fig. 11) configured for picking up containers from the storage 304. For clarity the storage 304 is omitted in Fig. 11 and therefore not visible. The gripping device 308 comprises a gripping member 310 which, in the present embodiment, comprises a pivotable gripping arm having three suction cups 311, thus enabling the gripping device 308 to grip and transport three containers simultaneously. For clarity the gripping member 310 is disclosed carrying only one container 100 in Fig. 11.

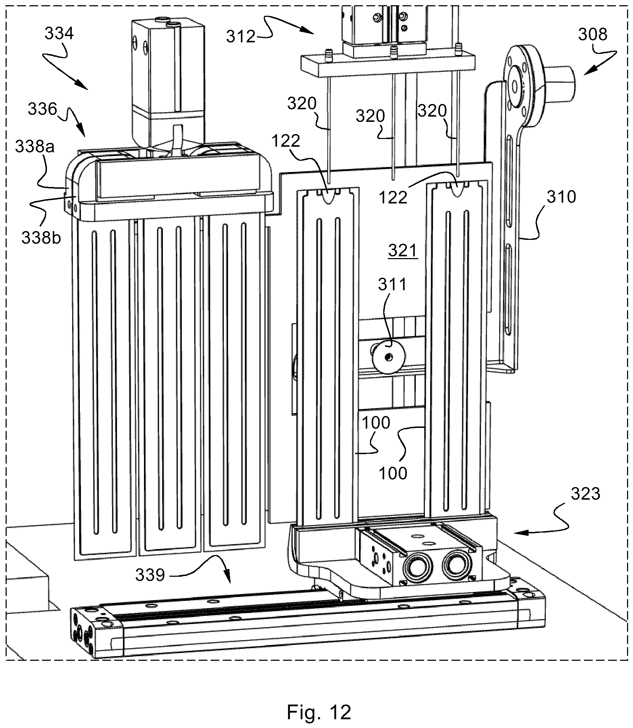

The apparatus 300 further comprises a filling station 312 (see Fig. 10). The filling station 312 is configured for filling the containers 100 with biological material to be cryopreserved. The filling station 312 comprises a storage, e.g. a tank (not shown), holding the biological material. The filling station 312 also comprises nozzles or cannulas 320 (see Fig. 12), each of which is configured for being inserted in a filling opening 122 in a container 100. The filling station 312 also comprises a pump (not shown) configured for supplying the biological material to the cannulas 320 from the storage.

As is illustrated in Fig. 12, the gripping arm 310 is configured to pick empty containers from the container storage 302 and by a rotating movement bring them into contact with a support surface 321 in the filling station 312. A holding arrangement 323 comprising clamps is configured for receiving the containers from the gripping arm 310. Once the containers 100 have been clamped by the holding arrangement 323, the cannulas 320 are introduced in the openings 122 and the containers 100 are filled. The filling station 312 is configured to fill three containers 100 in parallel. However, for clarity only two containers 100 are shown in Fig. 12.

The apparatus 300 further comprises a sealing station 334 configured for sealing the containers 100 after they have been filled with the biological material in the filling station 312. In the present embodiment, the sealing station 334 comprises a sealing device 336 (see Fig. 12) having sealing members or sealing clamps 338a, 338b which are configured to be brought into contact with the container to seal the opening 122 through which the biological material was filled (and possibly also openings through which air was evacuated from the containers during the filling operation). In the present embodiment, the sealing members 338a, 338b are configured to provide a weld or seam across the openings 122, 124, 126 by fusing the front 102 and back 104 walls in the area of the openings 122, 124, 126 (see Figs. 1-7), e.g. by pulse welding.

To bring the filled but not yet sealed containers from the filling station 312 to the sealing station 334, the apparatus 300 comprises a linear actuator 339 arranged to transport the holding arrangement 323 from the filling station 312 to the sealing station 334 in a translational movement. Once the suction cups 311 have released the containers and the linear actuator 339 has moved the filled containers from the filling station 312, the gripping arm 310 returns to the storage 304 to pick up a new set of containers for filling.

The apparatus 300 also comprises a racking station 340 configured for racking the filled and sealed containers 100 in racks 342 (see Fig. 10). In the present embodiment, the racking operation is accomplished using a robotic arm 343 which is configured to pick the sealed containers from the sealing station 334 and position them in the racks 342. In the present embodiment, each rack 342 holds approximately 16 containers.

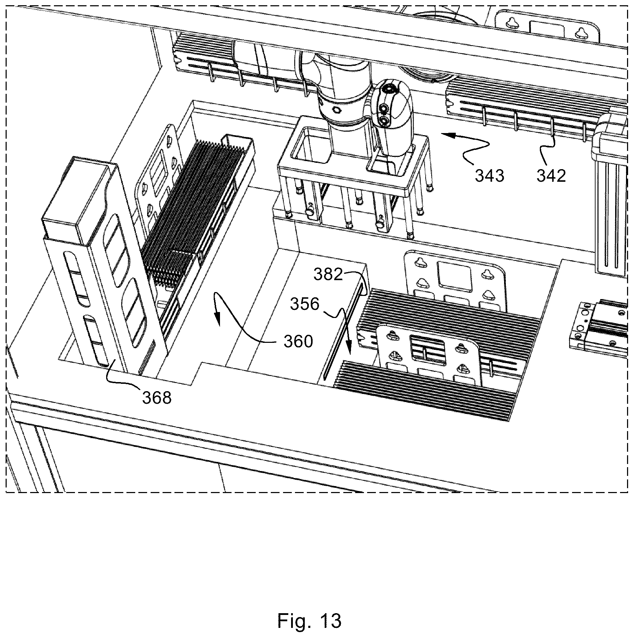

The apparatus 300 further comprises a freezing station 354 configured for bringing the biological material enclosed in the sealed containers to a cryogenic temperature, i.e. to a temperature below -180°C. The freezing station 354 comprises a first reservoir 356 holding a first freezing agent or fluid at a first, non-cryogenic temperature T1, and a second reservoir 360 holding a second freezing agent or fluid at a second, cryogenic temperature T2 (see also Fig. 13). The robotic arm 343 also functions as a rack conveyor configured for:
- bringing a rack 342 and the rack-mounted containers from the racking station 340 to the first reservoir 356 and immersing the rack-mounted containers in the first freezing agent;
- after a first period of time t1, lifting the rack and the rack mounted containers from the first reservoir 356 and disinvolving the rack-mounted containers from the first freezing agent;
- bringing the rack and the rack-mounted containers to the second reservoir 360 and immersing the rack-mounted containers in the second freezing agent; and
- after a second period of time t2, lifting the rack and the rack mounted containers from the second reservoir 360 and disinvolving the rack-mounted containers from the second freezing agent.

As previously discussed, the first freezing agent may be a freezing liquid kept at a constant or substantially constant temperature within the range of -20°C to -90°C, and the first period of time t1, i.e. the period of time the rack-mounted containers are kept immersed in the first freezing agent, may be within the range of 60s to 300s.

According to an alternative embodiment, the first freezing agent may be a freezing liquid kept at a continuously decreasing temperature within the range of +25°C to -90°C, preferably within ±0°C to -90°C, and the first period of time t1 may be within the range of 60s to 3600s.

The first freezing liquid may, according to one embodiment, be an oil. According to another embodiment, the first freezing liquid may be an alcohol, e.g. a linear and branched monohydric aliphatic C1 to C6 alcohol or a mixture thereof.

According to one embodiment, the second freezing agent may be liquid nitrogen and/or nitrogen vapour.

The second period of time t2, i.e. the period of time the rack-mounted containers are kept immersed in the second freezing agent, may be shorter, equal to or longer than the first period of time t1. However, in order to ensure that the biological material enclosed in the containers 100 are frozen to a cryogenic temperature, the second period of time t2 should preferably be at least 10 s, more preferably at least 30 s, more preferably at least 60 s, and even more preferably at least 120 s. Typically, the second period of time t2 may be within the range of 10 s to 240 s.

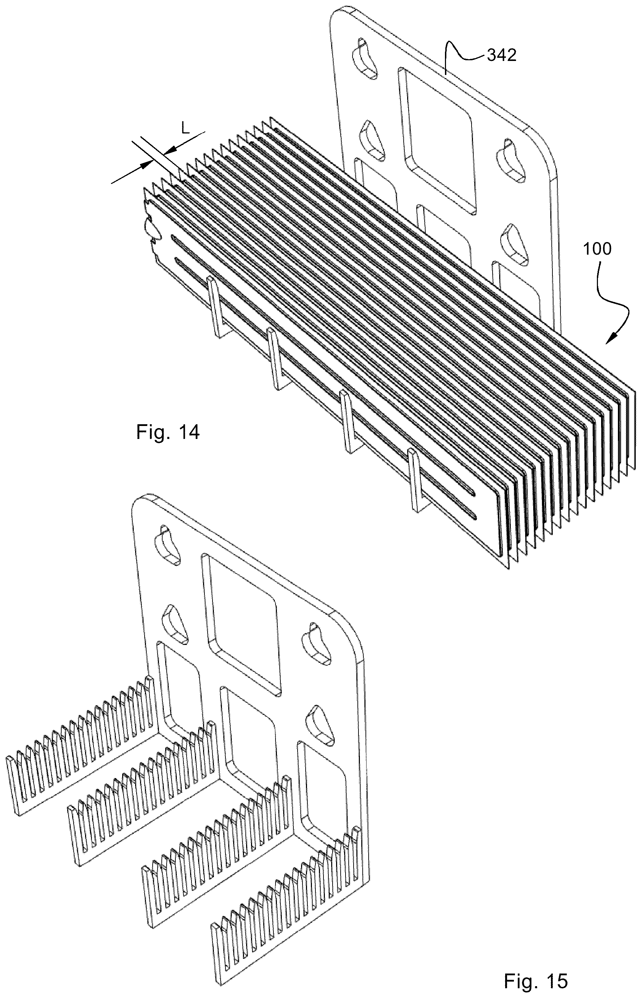

In the racking station, the containers 100 are stacked in the racks 342 in parallel planes, e.g. in a side-by-side configuration in parallel and vertical stacking planes with the longitudinal sides 114, 116 (see Figs. 1-7) of the containers 100 in a horizontal orientation, as is most clearly illustrated in Fig. 14. Advantageously, the distance L between adjacent stacking planes is at least 5 mm, preferably at least 10 mm, thus allowing the freezing agents in the first 356 and second 360 containers immediate access to the outside surfaces of the front and back walls 102, 104 of each container 100 (see Figs. 1-7), thereby ensuring efficient heat transfer from each container and the biological material enclosed therein. However, as previously discussed, in some embodiments the mutual distance between the racked containers may not exceed 15 mm.

As previously stated, each rack 342 comprises approximately 16 containers, thus allowing batch-wise freezing of containers 100.

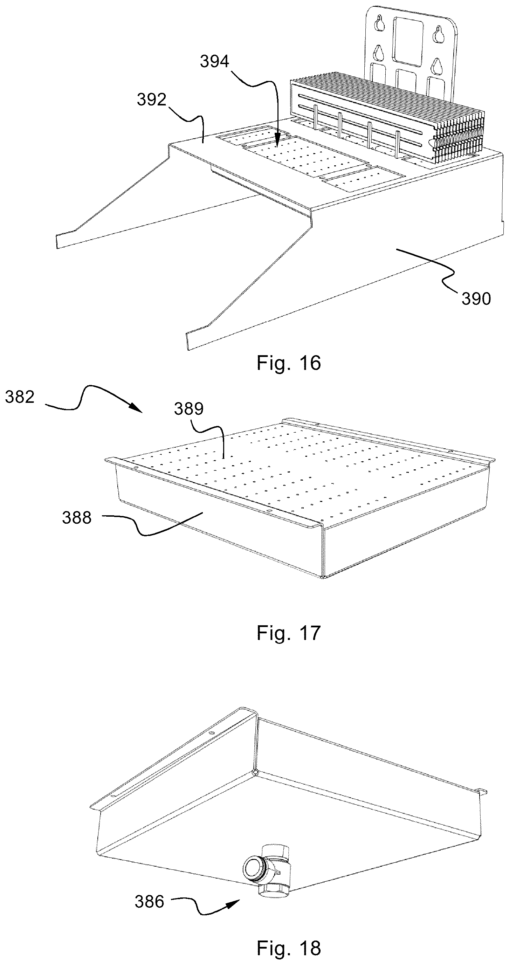

In the first reservoir 356, the first freezing agent is circulated between the racked containers 100. To this end, the apparatus 300 comprises a pump 380 (schematically illustrated in Fig. 10), a fluid distribution arrangement 382 (see Figs. 16-18) and a spillway opening 384 (see Fig. 13). The pump 380 is fluidly connected to the spillway opening 384 and to the fluid distribution arrangement 382 to circulate the first freezing agent from the spillway opening to the distribution arrangement 382.

The distribution arrangement 382 comprises an inlet 386 connected to the pump 380 and a fluid distribution box 388 (see Figs. 16-18). The fluid distribution box 388 is mounted in a stand 390 arranged at the bottom of the first reservoir 356. The stand 390 comprises a substantially horizontal surface 392 displaying through-openings 394 defining rack positions. The distribution box 388 comprises outlet openings 389 which are aligned with the through-openings 394 in the stand 390, thus allowing fluid received by the fluid distribution box 388 from the pump 380 via inlet 386 to be distributed underneath the racked containers via the outlet openings394.

In Fig. 16, a first rack position is occupied by a rack comprising racked containers, while a second rack position is illustrated without a landed rack for illustrative reasons. It is understood, however, that a rack with racked containers may also be landed on the empty rack position, thus allowing, in this embodiment, two racks with racked containers to be treated in parallel in the first reservoir 356.

Fluid exiting the outlet openings 389 will flow between the racked containers and, eventually, be led back to the pump 380 via spillway opening 384.

When disinvolving the rack-mounted containers from the first freezing agent (after having been immersed in the first freezing agent in the first reservoir during said first period of time, t1), the rack 342 may advantageously be raised from the first reservoir 356 at a vertical speed not exceeding 2 cm/s, and preferably not exceeding 1 cm/s. As previously discussed, this will allow the surface tension of the first freezing agent to draw and retain any freezing agent droplets formed on the surfaces of the rack 342 and the containers 100 stacked therein, thereby mitigating the problem of the first freezing agent being transferred to the second reservoir 360 together with the rack 342 and the containers and contaminating the second freezing agent.

The apparatus 300 also comprises a packaging station 366 configured for packaging the cryogenically frozen containers in boxes or packages 368 for subsequent cryogenic transport and/or storage (see Fig. 10 and also Fig. 13). In the present embodiment, the robotic arm 343 also functions as a rack conveyor configured for packaging the cryogenically frozen containers. The robotic arm 343 may advantageously be configured to pick the containers from the rack whilst the rack and the containers are still submerged in the second freezing agent, e.g. nitrogen vapour, disinvolve the containers from the second freezing agent and position the containers in the package 368. Alternatively, the containers may be packed in the package 368 while being immersed in the nitrogen vapour.

The apparatus 300 may comprises an ID marking station (not shown) configured for marking the containers 100 with an ID marking or tag, e.g. a QR code. The ID tag may identify the individual container, its content, date and time of filling or other desirable information. The ID marking station may for example comprise a printing device (not shown) configured for printing the ID marking on the containers.

The apparatus 300 may also comprise an ID scanning station (not shown) configured for scanning the containers 100 for an ID tag, e.g. to verify the ID tag applied to the container in the ID marking station. The ID scanning station may for example comprises an optical scanning device (not shown) configured for scanning a container for an ID marking.

The ID marking station, if present, may be arranged at the filling station 312 and the printing device may be configured to print the ID marking on the container during the filling of the container. The ID scanning station, if present, may also be arranged at the filling station 312. Alternatively, the ID marking and/or ID scanning station(s) may be arranged at the sealing station 334.

The apparatus 300 further comprises a control unit 370 configured for controlling the various sub-systems of the apparatus, e.g. the above-discussed stations, which control unit advantageously comprises a central processing unit (CPU) and an input/output interface allowing an operator to monitor and/or interact with the apparatus 300, e.g. to input program instructions.

The apparatus 300 further comprise a cabinet 376 in which the sub-systems are enclosed, thus allowing the sub-systems to be kept at a controlled environment, e.g. at a controlled temperature within the range of +0°C to +10°C, preferably within the range of +3°C to +5°C. To.

The following clauses illustrate preferred and optional embodiments according to the present disclosure.

Clause 1. A method of cryopreserving biological material (218) in a plurality of dimensionally stable containers (100), each container (100) comprising two substantially parallel walls (102, 104) connected to each other along a substantially part of their periphery and defining between them a storage space (134) having a substantially uniform thickness (D), the method comprising the steps of:
- at least partially filling the storage space (134) with the biological material via an opening (122) in the containers (100);
- closing the opening (122) enclosing the biological material (218) in the containers (100); and
- freezing the biological material (218) enclosed in the containers to a cryogenic temperature;
characterised by racking the containers (100) in substantially parallel planes and, during said step of freezing the biological material (218), bringing the racked containers (100) and the biological material enclosed therein to a first reservoir (256, 356) holding a first freezing agent (258) at a first temperature (T1) and fully immersing the racked containers (100) in the first freezing agent and circulating the first freezing agent (258) between the racked containers (100).

Clause 2. The method according to clause 1, wherein the step of racking the containers (100) comprises racking the containers (100) in substantially vertical planes.

Clause 3. The method according to any one of the preceding clauses, wherein said step of racking the containers (100) comprises racking the containers with a mutual distance of at least 5 mm.

Clause 4. The method according to any one of the preceding clauses, wherein said step of racking the containers (100) comprises racking the containers with a mutual distance within the range of 5 to 15 mm.

Clause 5. The method according to any one of the preceding clauses, comprising:
- keeping the racked containers (100) submerged in the first freezing agent (258) for a first period of time (t1);
- disinvolving the racked containers (100) from the first freezing agent (258);
- bringing the racked containers (100) from the first reservoir (256, 356) to a second reservoir (260, 360) holding a second freezing agent (262) at a second temperature (T2) being lower than the first temperature (T1) and immersing the racked containers (100) in the second freezing agent; and
- keeping the racked containers (100) submerged in the second freezing agent (262) for a second period of time (t2).

Clause 6. The method according to clause 5, wherein the first temperature (T1) is a non-cryogenic temperature, and wherein the second temperature (T2) is a cryogenic temperature.

Clause 7. The method according to any one of clauses 5 and 6, wherein the first period of time (t1) is within the range of 60s to 300s.

Clause 8. The method according to clause 7, comprising keeping the first temperature (T1) within the range of -20°C to -90°C during the first period of time (t1).

Clause 9. The method according to clause 8, wherein the first temperature (T1) is kept constant during the first period of time (t1).

Clause 10. The method according to any one of clauses 5 and 6, wherein the first period of time (t1) is within the range of 60s to 3600s.

Clause 11. The method according to clause 10, comprising keeping the first temperature (T1) within any one of the range of +25°C to -90°C and the range of ±0°C to -90°C during the first period of time (t1).

Clause 12. The method according to clause 11, wherein the first temperature (T1) is arranged to continuously decrease during the first period of time (t1).

Clause 13. The method according to any one of the preceding clauses, wherein the first freezing agent (258) is liquid alcohol.

Clause 14. The method according to any one of clauses 5-13, wherein the second temperature (T2) is below -180°C.

Clause 15. The method according to any one of clauses 5-14, wherein the second freezing agent (262) is liquid nitrogen and/or nitrogen vapour.

Clause 16. The method according to any one of the clauses 5-15, wherein the second period of time (t2) is any one of: longer than the first period of time (t1); shorter than the first period of time (t1); and equal to the first period of time (t1).

Clause 17. The method according to any one of clauses 5-16, wherein the second period of time (t2) is any one of: at least 10 s; at least 30 s; at least 60 s; at least 120 s; and within the range of 10 s to 240 s .

Clause 18. The method according to any one of the preceding clauses, wherein the biological material (208) comprises spermatozoa collected from an aquatic species.

Clause 19. The method according to any one of the preceding clauses, comprising, after the step of freezing the biological material (218), packaging the containers (100) in boxes or packages (368) for subsequent cryogenic transport and/or storage.

Clause 20. A cryopreservation apparatus (200, 300) for batch-wise cryopreservation of biological material (218) in dimensionally stable containers (100), each container comprising two substantially parallel walls (102, 104) connected to each other along a substantially part of their periphery (108) and defining between them a storage space (134) having a substantially uniform thickness (D), the apparatus comprising:
- a filling station (212, 312) configured for filling the containers (100) with the biological material (218);
- a sealing station (234, 343) configured for sealing the containers (100) enclosing the biological material (218) in the containers (100); and
- a freezing station (254, 354) configured for freezing the biological material (218) enclosed in the containers (100) to a cryogenic temperature,
characterised in that the apparatus (200, 300) comprises a racking station (240, 340) arranged downstream of the sealing station (234, 334) and upstream of the freezing station (254, 354) for racking a plurality of said containers (100) in a rack (242, 342) in substantially parallel planes, and in that the freezing station (254, 354) comprises:
- a first reservoir (256, 356) configured for holding a first freezing agent (258) at a first temperature (T1) and for receiving the racked containers (100) fully immerged in the first freezing agent (258); and
- a rotor (280) or pump (380) configured for circulating the first freezing agent (258) between the immersed, racked containers.

Clause 21. The apparatus (200, 300) according to clause 20, characterised in that the racking station (240, 340) is configured to rack the containers (100) in substantially vertical planes.

Clause 22. The apparatus (200, 300) according to any one of clauses 20 and 21, characterised in that the racking station (240, 340) is configured to rack the containers (100) with a mutual distance (L) of at least 5 mm.

Clause 23. The apparatus (200, 300) according to any one of clauses 20-22, characterised in that the racking station (240, 340) is configured to rack the containers (100) with a mutual distance (L) within a range of 5 to 15 mm.

Clause 24. The apparatus (200, 300) according to any one of clauses 20-23, characterised in that the first reservoir (256, 354) comprises a fluid distributor arrangement (382) configured for evenly distributing the circulated first freezing agent (258) between the racked containers (100).

Clause 25. The apparatus (200, 300) according to any one of clauses 20 and 21, characterised in that the freezing station (254, 354) comprises a second reservoir (260, 360) configured for holding a second freezing agent (262) at a second temperature (T2) being lower than the first temperature (T1), the second reservoir (260, 360) being configured for receiving the racked containers (100) fully immerged in the second freezing agent (262) after the racked containers (100) first having been fully immerged in the first freezing agent (258, 360).

Clause 26. The apparatus (200, 300) according to clause 25, characterised in that the first temperature (T1) is a non-cryogenic temperature and the second temperature (T2) is a cryogenic temperature.

Clause 27. The apparatus (200, 300) according to any one of clauses 20-26, characterised in that the first freezing agent (258) is liquid alcohol.

Clause 28. The apparatus (200, 300) according to any one of clauses 25-27, characterised in that the second freezing agent (262) is liquid nitrogen and/or nitrogen vapour.

Clause 29. The apparatus (200, 300) according to any one of clauses 20-28, characterised in that it comprises a packaging station (266, 366) arranged downstream of the freezing station (254, 354), the packaging station (266, 366) being configured for packaging the cryogenically frozen containers in boxes or packages (368) for subsequent cryogenic transport and/or storage.

It is appreciated that certain features of the invention, which, for clarity, have been described above in the context of separate embodiments, may also be provided in combination in a single embodiment. Conversely, various features of the invention, which, for brevity, have been described in the context of a single embodiment, may also be provided separately or in any suitable sub-combination. In particular, it will be appreciated that features described in relation to one particular embodiment may be interchangeable with features described in relation to the other embodiments.

In the preceding description, various aspects of the apparatus according to the invention have been described with reference to the illustrative embodiment. For purposes of explanation, specific numbers, systems and configurations were set forth in order to provide a thorough understanding of the apparatus and its workings. However, this description is not intended to be construed in a limiting sense. Various modifications and variations of the illustrative embodiment, as well as other embodiments of the apparatus, which are apparent to person skilled in the art to which the disclosed subject-matter pertains, may lie within the scope of the present invention as defined by the following claims.

## Claims

1. A method of cryopreserving biological material (218) in a plurality of dimensionally stable containers (100), each container (100) comprising two substantially parallel walls (102, 104) connected to each other along a substantially part of their periphery and defining between them a storage space (134) having a substantially uniform thickness (D), the method comprising the steps of, in a housing or cabinet of a cryopreservation apparatus:
- at least partially filling the storage space (134) with the biological material via an opening (122) in the containers (100);
- closing the opening (122) enclosing the biological material (218) in the containers (100);
- racking the containers (100) in substantially parallel planes; and
- freezing the biological material (218) enclosed in the containers to a cryogenic temperature;
wherein said step of freezing the biological material (218) comprises bringing the racked containers (100) and the biological material enclosed therein to a first reservoir (256, 356) holding a first freezing agent (258) at a first temperature (T1) and fully immersing the racked containers (100) in the first freezing agent and circulating the first freezing agent (258) between the racked containers (100).

2. The method according to claim 1, wherein the step of racking the containers (100) comprises racking the containers (100) in substantially vertical planes.

3. The method according to any one of the preceding claims, wherein said step of racking the containers (100) comprises racking the containers with a mutual distance of at least 5 mm.

4. The method according to any one of the preceding claims, wherein said step of racking the containers (100) comprises racking the containers with a mutual distance within the range of 5 to 15 mm.

5. The method according to any one of the preceding claims, comprising:
- keeping the racked containers (100) submerged in the first freezing agent (258) for a first period of time (t1);
- disinvolving the racked containers (100) from the first freezing agent (258);
- bringing the racked containers (100) from the first reservoir (256, 356) to a second reservoir (260, 360) holding a second freezing agent (262) at a second temperature (T2) being lower than the first temperature (T1) and immersing the racked containers (100) in the second freezing agent; and
- keeping the racked containers (100) submerged in the second freezing agent (262) for a second period of time (t2).

6. The method according to claim 5, wherein the first temperature (T1) is a non-cryogenic temperature, and wherein the second temperature (T2) is a cryogenic temperature.

7. The method according to any one of claims 5 and 6, wherein the first period of time (t1) is within the range of 60s to 300s.

8. The method according to claim 7, comprising keeping the first temperature (T1) within the range of -20°C to -90°C during the first period of time (t1).

9. The method according to claim 8, wherein the first temperature (T1) is kept constant during the first period of time (t1).

10. The method according to any one of claims 5 and 6, wherein the first period of time (t1) is within the range of 60s to 3600s.

11. The method according to claim 10, comprising keeping the first temperature (T1) within any one of the range of +25°C to -90°C and the range of ±0°C to -90°C during the first period of time (t1).

12. The method according to claim 11, wherein the first temperature (T1) is arranged to continuously decrease during the first period of time (t1).

13. The method according to any one of the preceding claims, wherein the first freezing agent (258) is liquid alcohol.

14. The method according to any one of claims 5-13, wherein the second temperature (T2) is below -180°C.

15. The method according to any one of claims 5-14, wherein the second freezing agent (262) is liquid nitrogen and/or nitrogen vapour.

16. The method according to any one of the claims 5-15, wherein the second period of time (t2) is any one of: longer than the first period of time (t1); shorter than the first period of time (t1); and equal to the first period of time (t1).

17. The method according to any one of claims 5-16, wherein the second period of time (t2) is any one of: at least 10 s; at least 30 s; at least 60 s; at least 120 s; and within the range of 10 s to 240 s .

18. The method according to any one of the preceding claims, wherein the biological material (208) comprises spermatozoa collected from an aquatic species.

19. The method according to any one of the preceding claims, comprising, after the step of freezing the biological material (218), packaging the containers (100) in boxes or packages (368) for subsequent cryogenic transport and/or storage.

20. A cryopreservation apparatus (200, 300) for batch-wise cryopreservation of biological material (218) in dimensionally stable containers (100), each container comprising two substantially parallel walls (102, 104) connected to each other along a substantially part of their periphery (108) and defining between them a storage space (134) having a substantially uniform thickness (D), the apparatus comprising:
- a filling station (212, 312) configured for filling the containers (100) with the biological material (218);
- a sealing station (234, 343) configured for sealing the containers (100) enclosing the biological material (218) in the containers (100); and
- a freezing station (254, 354) configured for freezing the biological material (218) enclosed in the containers (100) to a cryogenic temperature,
**characterised in that** the apparatus (200, 300) comprises a racking station (240, 340) arranged downstream of the sealing station (234, 334) and upstream of the freezing station (254, 354) for racking a plurality of said containers (100) in a rack (242, 342) in substantially parallel planes, and **in that** the freezing station (254, 354) comprises:
- a first reservoir (256, 356) configured for holding a first freezing agent (258) at a first temperature (T1) and for receiving the racked containers (100) fully immerged in the first freezing agent (258); and
- a rotor (280) or pump (380) configured for circulating the first freezing agent (258) between the immersed, racked containers,
wherein the cryopreservation apparatus comprises a housing or cabinet in which the filling station, the sealing station, the racking station and the freezing station are housed.

21. The apparatus (200, 300) according to claim 20, **characterised by** a packaging station (266, 366) arranged downstream of the freezing station (254, 354), the packaging station (266, 366) being configured for packaging the cryogenically frozen containers in boxes or packages (368) for subsequent cryogenic transport and/or storage.

22. The apparatus (200, 300) according to claim 21, **characterised by** the packaging station (266, 366) being housed in said housing or cabinet.

23. The apparatus (200, 300) according to any one of claims 20-22, **characterised in that** the racking station (240, 340) is configured to rack the containers (100) in substantially vertical planes.

24. The apparatus (200, 300) according to any one of claims 20-23, **characterised in that** the racking station (240, 340) is configured to rack the containers (100) with a mutual distance (L) of at least 5 mm.

25. The apparatus (200, 300) according to any one of claims 20-24, **characterised in that** the racking station (240, 340) is configured to rack the containers (100) with a mutual distance (L) within a range of 5 to 15 mm.

26. The apparatus (200, 300) according to any one of claims 20-25, **characterised in that** the first reservoir (256, 354) comprises a fluid distributor arrangement (382) configured for evenly distributing the circulated first freezing agent (258) between the racked containers (100).

27. The apparatus (200, 300) according to any one of claims 20-26, **characterised in that** the freezing station (254, 354) comprises a second reservoir (260, 360) configured for holding a second freezing agent (262) at a second temperature (T2) being lower than the first temperature (T1), the second reservoir (260, 360) being configured for receiving the racked containers (100) fully immerged in the second freezing agent (262) after the racked containers (100) first having been fully immerged in the first freezing agent (258, 360).

28. The apparatus (200, 300) according to claim 27, **characterised in that** the first temperature (T1) is a non-cryogenic temperature and the second temperature (T2) is a cryogenic temperature.

29. The apparatus (200, 300) according to any one of claims 20-28, **characterised in that** the first freezing agent (258) is liquid alcohol.

30. The apparatus (200, 300) according to any one of claims 25-29, **characterised in that** the second freezing agent (262) is liquid nitrogen and/or nitrogen vapour.
